# NEUE EUROPÄISCHE PATENTSCHRIFT

(11) **EP 3 046 593 B2**
(45) Veröffentlichungstag und Bekanntmachung des Hinweises auf die Entscheidung über den Einspruch: **20.05.2026**
(45) Hinweis auf die Patenterteilung: 22.07.2020
(21) Anmeldenummer: 14781462.8
(22) Anmeldetag: 12.09.2014
(51) Int. Cl.: A61L 29/16

(54) **LIMUS-DEPOT-FORMULIERUNG AUF BALLONKATHETERN**
LONG-ACTING LIMUS FORMULATION ON BALLOON CATHETERS
FORMULATION DE DÉPÔT DE LIMUS SUR DES CATHÉTERS À BALLONNET

(30) Priorität: 18.09.2013 DE 102013110294
(43) Veröffentlichungstag der Anmeldung: 27.07.2016
(73) Patentinhaber: Innora GmbH, 10115 Berlin (DE)
(72) Erfinder: PETERS, Daniel, 12109 Berlin (DE)
(74) Vertreter: ETL IP Patent- und Rechtsanwaltsgesellschaft mbH
(86) Internationale Anmeldenummer: PCT/EP2014/069517
(87) Internationale Veröffentlichungsnummer: WO 2015/039969

(56) Entgegenhaltungen:
- EP-A1- 2 944 334
- EP-A1- 3 007 679
- WO-A1-00/32238
- WO-A1-2011/131678
- WO-A1-2013/012689
- WO-A1-2013/091722
- WO-A2-2009/018816
- WO-A2-2010/086863
- WO-A2-2010/124098
- WO-A2-2011/005421
- WO-A2-2011/008393
- US-A1- 2010 015 200
- US-A1- 2011 008 260
- US-A1- 2011 008 260
- US-A1- 2011 015 664
- US-A1- 2011 130 829
- US-A1- 2011 238 011
- US-A1- 2012 015 019
- US-A1- 2012 172 787
- US-A1- 2012 231 037
- US-A1- 2013 035 483
- US-A1- 2013 053 947
- US-A1- 2013 280 315

## Beschreibung

Binnen weniger Wochen oder Monate nach der Wiedereröffnung verengter oder verschlossener Arterien und anderer Passagen im Körper mittels verschiedener mechanischer oder thermischer Verfahren kommt es häufig zu einer erneuten Verengung aufgrund überschießender Gewebeproliferation. Dieser Vorgang und seine Verhinderung ist besonders sorgfältig an den Koronararterien untersucht worden. Seit etwa 2002 werden arzneimittelfreisetzende Stents implantiert, die das Lumen der Koronarien nach Erweiterung auf den ursprünglichen Durchmesser nicht nur durch ausreichende Radialkraft offenhalten sondern durch anhaltende Freisetzung proliferationshemmender Arzneistoffe auch das Wachstum von Gefäßwandbestandteilen durch die Stentstreben hindurch in das Lumen begrenzen.

Ursprünglich wurden 2 Substanzklassen erfolgreich zur Beschichtung der Stents eingesetzt, Macrolidlactone wie Rapamycin (= Sirolimus), Everolimus, Biolimus, Zotarolimus, die an mTOR (mammalian target of rapamycin) binden und dadurch die Zellteilung hemmen, sowie das Taxan Paclitaxel, das Microtubuli stabilisiert und ebenfalls die Zellteilung hemmt. Seither haben sich die Macrolidlactone, auch als Limus-Substanzen bezeichnet, bei der Beschichtung von Stents durchgesetzt.

In Ergänzung zu den koronaren Stents und als Alternative zur Behandlung anderer Arterien sind inzwischen arzneimittelbeschichtete Ballonkatheter verfügbar. Wie beim Stent dient der Arzneistoff der Verhinderung der Wiederverengung der mittels Ballonangioplastie erweiterten Gefäße. Allerdings wird der Arzneistoff vom Ballon nur während der kurzen Zeit der Ballonexpansion (< 1min bis max 5 min in peripheren Gefäßen) abgegeben. Im Gegensatz zu den Stents ist Paclitaxel der dominierende Wirkstoff. Limus-Substanzen sind seit Jahren vielfach tierexperimentell untersucht worden, aber bisher mit unbefriedigenden, widersprüchlichen oder schlecht reproduzierbaren Ergebnissen (Cremers B, Toner JL, Schwartz LB, von Oepen R, Speck U, Kaufels N, Clever YP, Mahnkopf D, Böhm M, Scheller B. Inhibition of Neointimal Hyperplasia with a Novel Zotarolimus Coated Balloon Catheter. Clin Res Cardiol. 2012; 101: 469-76; US20100331816; Takimura CK, Galon MZ, Sojitra P, Doshi M, Aiello V, Gutierrez PS, Carvalho J, Ferreira SK, Chaves MJF, Laurindo FRM, Lemos PA. Estudo da Dose Excipiente:Färmaco com Avaliação da Hiperplasia Neointimal por Tomografia de Coerencia Óptica e Histopatologia em Arterias Coronárias Porcinas após o Emprego do Baläo Eluidor de Sirolimus. Rev Bras Cardiol Invasiva. 2012;20(2):133-9; Schmehl J, von der Ruhr J, Dobratz M, Kehlbach R, Braun I, Greiner T-O, Claussen CD, Behnisch B. Balloon Coating with Rapamycin Using an On-site Coating Device. Cardiovasc Intervent Radiol 2013;36:756-763; Granada JF, Milewski K, Zhao H, Stankus JJ, Tellez A, Aboodi MS, BS; Kaluza GL, Krueger CG, Virmani R, MD; Schwartz LB, Nikanorov A. Vascular Response to Zotarolimus-Coated Balloons inlnjured Superficial Femoral Arteries of the Familial Hypercholesterolemic Swine Circ Cardiovasc Interv. 2011;4:447-455). Ein klinischer Wirkungsnachweis im Hinblick auf eine Hemmung der Restenose liegt bisher nicht vor.

Die Ursache für die geringe Wirksamkeit von Limus-Substanzen auf Ballonkathetern wird in dem geringen Transfer der Arzneistoffe in die Gefäßwand und der Tatsache gesehen, dass die Wirkstoffspiegel in der Gefäßwand nicht lange genug aufrechterhalten werden, um die erwünschte Wirkung zu verursachen (Gray WA, Granada JF. Drug-coated balloons for the prevention of vascular restenosis. Circulation. 2010 Jun 22;121(24):2672-2680, see pages 2673-2674, figure 1; Tellez A, Buszman P, Afari M, Palmieri T, Cheng Y, Rate W, Stone S, Conditt G, Keng Y-F, Bingham B, Baumbach W, Sherman D, Kaluza G, Granada J. Acute Delivery and Long Term Retention of Sirolimus Nanoparticles Using a Novel Porous Angioplasty Balloon in the Porcine Coronary Model. JACC 2012; 60/17/Suppl B: B173).

Verglichen mit Paclitaxel ist es gelungen initial ähnliche Wirkstoffkonzentrationen in der Arterienwand zu erreichen, allerdings fällt die Wirkstoffkonzentration wesentlich schneller ab, so dass die Konzentrationen später wesentlich niedriger sind.

Von Limus-Substanzen sind wie von vielen anderen Arzneistoffen sowohl amorphe (z.B. WO2006039237 A1, WO2010129328 A1) als auch kristalline Formen bekannt. Beide Formen haben bestimmte Vor- und Nachteile je nach Anwendung.

Es ist zum Beispiel aus der US 2013/053 947 A1 bekannt, dass sich Kristalle von Wirkstoffen, speziell auch von Limus-Wirkstoffen langsamer auflösen als die amorphe Substanz. Dies ist genutzt worden, um die Freigabe von Rapamycin von Stentoberflächen zu verzögern. Ein kompliziertes Verfahren wurde beschrieben, um Stents mit geeigneten Rapamycin-Kristallen zu beschichten (Farah S, Khan W, Domb AJ. Crystalline coating of rapamycin onto a stent: Process development and characterization. Int J Pharmaceutics 2013;445:20-28).

Die Untersuchungen zur Stentbeschichtung sind nicht einfach auf die Beschichtung von Angioplastie-Ballonen übertragbar. Der Stent wird in die Arterie eingebracht und verbleibt dort. Die Substanz kann sich im Falle des Stents von einer stabilen Oberfläche mit mehreren Lagen von Kristallen langsam ablösen. Der Ballon kommt dagegen nur für sehr kurze Zeit mit der Gefäßwand in Kontakt. Dabei muß die notwendige Dosis in die Gefäßwand übertreten.

Sofern der Wirkstoff nicht bereits gelöst ist erfolgt die Auflösung einzelner Partikel oder Kristalle, die von allen Seiten für das Lösungsmittel zugänglich sind.

Die Verwendung von Kristallen zur Beschichtung von Ballonkathetern ist wegen des Embolierisikos umstritten und wird z. T. abgelehnt. Amorphe Beschichtungen werden bevorzugt (WO 2011/147408, S. 4, Zeilen 14-24).

Aufgabe der vorliegenden Erfindung ist es, Ballone von Ballonkathetern in einer Weise mit Limus-Substanzen, zu beschichten, dass die Beschichtung ausreichend haftet, um auf dem Weg zum verengten Arteriensegment nicht verloren zu gehen, sich bei Expansion des Ballons weitgehend vollständig abzulösen, zu einem ausreichenden Anteil in die Gefäßwand überzutreten und dort lange genug zu verweilen, um anhaltend wirksam zu werden. Maß für die Erreichung dieser Ziele sind einerseits ähnliche Eigenschaften wie diejenigen der als klinisch wirksam bekannten Paclitaxel-Beschichtungen und andererseits deutlich länger anhaltende hohe Wirkstoffkonzentrationen im Gewebe als sie für die Macrolidlactone, insbesondere Limus-Substanzen, bisher bekannt sind.

Die Aufgabe wird gelöst mit einem Beschichtungsverfahren gemäß Anspruch 1. Weitere bevorzugte Ausführungsformen ergeben sich aus dem abhängigen Anspruch.

Das erfindungsgemäße Verfahren führt zu Ballonkathetern mit einer Beschichtung auf der Ballonoberfläche aufweisend mindestens eine Limus-Substanz in nicht-verkapselter kristalliner Form. Dabei ist vorgesehen, dass die kristalline nicht-verkapselte Limus-Substanz direkt aus einem Lösungsmittelgemisch aus mindestens einem polaren organischen Lösungsmittel und mindestens einem unpolaren organischen Lösungsmittel aufgetragen wird.

Der Begriff "Ballonkatheter" bedeutet wie im üblichen Sinne Angioplastie-Ballonkatheter, d.h. Ballonkatheter für die perkutane transluminale Angioplastie zur Erweiterung oder Wiedereröffnung von verengten oder verschlossenen Blutgefäßen (meistens Arterien, seltener auch Venen) mittels Ballondilatation. Beschichtungen für Ballonkatheter müssen auf dem Weg zum verengten bzw. verschlossenen Segment eines Blutgefäßes an dem Ballon haften, d.h. während dieser durch ein hämostatisches Ventil geführt wird sowie auf dem Weg durch eine mit Blut gefüllte Einführschleuse bzw. einen Führungskatheter und durch proximale Abschnitte des Blutgefäßes, und den Wirkstoff dann beim Füllen des Ballons schnell an die Gefäßwand abgeben. Der Ballonkatheter verbleibt nach der Operation im Gegensatz zu einem Implantat wie einem Stent oder einem implantierbaren oder Verweil-Katheter nicht im Körper. "Mindestens eine Limus-Substanz" bedeutet, dass auch Mischungen aus mehreren Limus-Subtanzen umfasst sind. Vorzugsweise wird eine einzelne Limus-Substanz eingesetzt.

Die Limus-Substanzen (synonym Limus-Arzneistoffe) sind bevorzugt ausgewählt aus Sirolimus, Everolimus, Zotarolimus, Biolimus, Temsirolimus, Myolimus, Novolimus, Ridaforolimus sowie Tacrolimus und Pimecrolimus. Mehr bevorzugt ist die Gruppe Sirolimus, Everolimus, Zotarolimus, Biolimus, Temsirolimus. Die besonders bevorzugte Gruppe besteht aus Sirolimus und Everolimus. Als Limus-Substanz wird höchst bevorzugt Everolimus eingesetzt. Alternativ wird Sirolimus höchst bevorzugt.

Die oben genannten Ziele werden erfindungsgemäß überraschend gut, vollständig und reproduzierbar und wirtschaftlich erreicht: Limus-Arzneistoffe werden auf an sich bekannte Weise in geeigneten Lösungsmitteln zur Kristallisation gebracht. Um eine ausreichende Dosierung auf den Ballonoberflächen zu erzielen werden Lösungsmittelgemische aus mindestens einem polareren organischen Lösungsmittel und mindestens einem unpolareren organischen Lösungsmittel verwendet. Das unpolarere und das polarere organische Lösungsmittel weisen vorzugsweise mindestens eine Differenz ihres logK_{ow} von 1 auf (K_{ow}: Verteilungskoeffizient Oktanol/Wasser). Als polareres organisches Lösungsmittel wird insbesondere ein organisches Lösungsmittel mit einem logK_{ow} zwischen -1,0 und +2,0, vorzugsweise zwischen -0,5 und +1,8, verstanden. Als unpolarer werden insbesondere organische Lösungsmittel mit einem logK_{OW} ≥3, vorzugsweise zwischen 3 und 6,5, verstanden. Polarere organische Lösungsmittel werden synonym auch verkürzt als polarere Lösungsmittel bezeichnet, gleiches gilt für die unpolareren organischen Lösungsmittel (unpolarere Lösungsmittel). In mindestens einem der Lösungsmittel, vorzugsweise im organischen polareren Lösungsmittel, soll die Limus-Substanz eine Löslichkeit von > 10 mg/ml aufweisen, bevorzugt > 30 mg/ml. Beispiele sind flüchtige organische Lösungsmittel wie Alkohole, Aceton, Ethylactetat, Chloroform. Als Alkohole werden insbesondere ein- oder mehrwertige Alkanole, mehr bevorzugt einwertige C1-C3-Alkanole, höchst bevorzugt Methanol und/oder Ethanol, verstanden. Weitere polarere organische Lösungsmittel sind Tetrahydrofuran, Acetonitril, Diethylether. In dem anderen oder einem der anderen Lösungsmittel, vorzugsweise im unpolareren organischen Lösungsmittel, soll die Limus-Substanz nur wenig löslich sein, z. B. mit < 1mg/ml (0,001 bis 0,999 mg/ml). Beispiele für organische Lösungsmittel mit niedriger Löslichkeit für Limus-Substanzen wurden in US 20110009618 A1 genannt; es sind insbesondere sehr unpolare Lösungsmittel wie aliphatische C6-C10-Kohlenwasserstoffe, beispielsweise Cyclohexan, Hexan, Heptan, Octan etc.. Die Lösungsmittel oder Lösungsmittelgemische können Wasser enthalten, wie nachfolgend noch näher erläutert wird.

Bevorzugte Lösungen zur Kristallisation oder direkten Beschichtung enthalten 20-80 Volumen% eines polareren Lösungsmittels und 80-20 Volumen% eines unpolareren Lösungsmittels, besonders bevorzugt sind Mischungen aus 30-70 Volumen% eines der genannten polareren Lösungsmittel und 65-35 Volumen% eines der genannten unpolareren Lösungsmittel.

Der Begriff "unpolareres bzw. polareres organisches Lösungsmittel" umfasst auch Mischungen mehrerer Lösungsmittel einer und/oder beider Kategorien, vorzugsweise wird allerdings jeweils ein Lösungsmittel pro Kategorie eingesetzt. Ein bevorzugtes Paar aus polarerem und unpolarerem organischen Lösungsmittel ist beispielsweise Ethylacetat / Heptan.

Die Limus-Substanz kann zunächst in einem polareren organischen Lösungsmittel, z.B. Ethanol oder anderen Alkoholen, Aceton, Ethylacetat, Tetrahydrofuran, Acetonitril, Diäthylether etc gelöst werden (Schritt a), danach kann die Lösung mit dem unpolareren Lösungsmittel versetzt werden, so dass entweder eine übersättigte Lösung entsteht oder die echte Löslichkeit erhalten bleibt (Schritt b). Im Fall einer übersättigten Lösung kann die Kristallisation der Limus-Substanz durch geeignete Maßnahmen, beispielsweise durch Reiben von Glas auf Glas oder durch Kristallkeime ausgelöst werden (Schritt d) oder die übersättigte oder die echte Lösung wird ohne das Vorhandensein von Kristallen auf den Ballon aufgetragen und dort kristallisiert (Schritt c). Erfolgt die Kristallisation bereits in der Lösung, kann die Suspension auf die Ballonoberfläche aufgetragen und dort weiter kristallisiert und/oder getrocknet werden (Schritt d1).

Es gibt verschiedene erfindungsgemäße Möglichkeiten, Oberflächen eines Ballons eines Ballonkatheters reproduzierbar mit Limus-Kristallen zu beschichten:
a) Limus-Kristalle werden in einem Lösungsmittel oder Lösungsmittelgemisch, in dem sich die Kristalle nicht auflösen, suspendiert. Vorzugsweise werden hierbei als Lösungsmittel / Suspensionsmittel die oben genannten unpolareren organischen Lösungsmittel eingesetzt. Diese Suspension wird in therapeutischer Dosis auf den Ballon aufgetragen.
b) Limus-Kristalle werden wie unter (a) beschrieben, jedoch in sehr niedriger, subtherapeutischer Dosis, beispielsweise zwischen 0,001 und 0,5 µg Limus-Substanz/mm² Ballonoberfläche, bevorzugt zwischen 0,001 und 0,1 µg Limus-Substanz/mm², aufgetragen um Impfkristalle auf dem Ballon zu deponieren. Sofort danach oder nach Trocknen der Impfkristalle wird mit einer weitgehend oder vollständig gesättigten oder übersättigten Limus-Lösung beschichtet bis eine therapeutisch wirksame Dosis erreicht ist. In anderen Worten wird eine zusätzliche Dosis der mindestens einen Limus-Substanz in Form einer mindestens gesättigten Lösung aufgebracht, entsprechend einer Dosis zwischen 1 und 10 µg Limus-Substanz/ mm² Ballonoberfläche (im trockenen Endzustand). Gesättigte oder übersättigte Lösungen können in sehr unterschiedlichen Lösungsmitteln hergestellt werden, bevorzugt sind Lösungsmittelgemische aus einem polaren (Ethylacetat, Aceton, Isopropanol) und einem unpolaren Lösungsmittel (z.B. Cyclohexan, Hexan, Heptan, Octan), gegebenenfalls unter Zusatz von Wasser. Die Kristallisation, die Größe der Kristalle und deren Aggregation lässt sich über die Trocknungsbedingungen, insbesondere Temperatur und Luftbewegung steuern.

Erfindungsgemäß sind nicht verkapselte, auch nicht teilweise verkapselte freie Mikrokristalle (Im Unterschied zu Micell Technologies US2012015442, WO2013059509). Die (freien) Mikro-kristalle können sich in einer Matrix auf der Ballonoberfläche befinden, wobei die Matrix entweder die Haftung an der Ballonoberfläche fördert oder die Freisetzung der Wirkstoffkristalle bei Ballonexpansion, nicht jedoch die Freisetzung des Wirkstoffs aus der Kapsel nach Übertritt ins Gewebe.

Der Bereich therapeutisch wirksamer, d.h. die Neointimaproliferation hemmender oder anderweitig wirksamer Dosierungen liegt bevorzugt zwischen 1 und 10 µg Limus-Substanz/mm² Ballonoberfläche.

Mehr als 30 Gewichts% der Limus-Substanz sollte nach der Beschichtung in Form von Kristallen auf der Ballonoberfläche vorliegen, bevorzugt mehr als 50 Gew.-% und besonders bevorzugt mehr als 70 Gew.-%. Die Einzelkristalle, die so genannten Mikrokristalle, sind bevorzugt rhombisch in der Form und variabel in der Größe, wobei ein wesentlicher Teil der Kristalle (bezogen auf die Masse), d.h. > 30 Gew.-%, zwischen 1-300 µm als größte Längenausdehnung aufweisen kann, bevorzugt > 50 Gew.-%, mehr bevorzugt > 80 Gew.-%. Durch die Trocknung entstehen Aggregate aus Einzelkristallen, die größer sein können.

Der Schmelzpunkt der Kristalle liegt im Bereich von 171- 188 °C. Die Verweilzeit der von den Ballonen in das Gewebe übertragenen Limus-Substanzen ist gegenüber den bekannten Zubereitungen deutlich verlängert. Die mittlere Halbwertszeit (Eliminationshalbwertszeit) in Schweinekoronarien beträgt ≥ 1 Woche, bevorzugt ≥ 2 Wochen. In anderen Worten ist der Ballonkatheter mit einer Beschichtung auf der Ballonoberfläche aufweisend mindestens eine Limus-Substanz in kristalliner Form dadurch gekennzeichnet, dass die Kristalle nach Übertragung durch einen Ballonkatheter in Schweinekoronarien eine Eliminationshalbwertszeit von ≥ 1 Woche, bevorzugt ≥2 Wochen innerhalb des Zeitraums von 4 Wochen nach der Behandlung aufweisen.

Die Beschichtung kann ausschließlich die Limus-Substanz ggf. auch in Solvatkristallen enthalten. Der Beschichtung können unterschiedliche Hilfsstoffe und/oder Additive zugesetzt werden, bevorzugt sind Beschichtungen ohne Polymere, d.h. die Beschichtung ist bevorzugt polymerfrei. Geeignet als Hilfsstoffe / Additive sind unter anderem Antioxidantien, bevorzugt Ascorbylpalmitat, Butylhydroxyanisol, Butylhydroxytoluol, Nordihydroguaretsäure, Probucol, Propylgallat, Resveratrol, besonders bevorzugt Butylhydroxytoluol und/oder Resveratrol und am meisten bevorzugt Resveratrol; weiterhin geeignet sind andere zur Beschichtung von Arzneimitteln-freisetzenden Ballonkathetern verwendete hoch- und niedermolekulare Substanzen wie beispielsweise in USP 8,439,686, US2010324648, US 2008/0118544, USP 20130123695 genannt oder gebräuchliche pharmazeutische Hilfsstoffe. In anderen Worten besteht die Beschichtung in einer bevorzugten Ausführungsform nur aus der Limus-Substanz, ggf. auch in Solvatkristallen, und weiterhin optional aus Hilfsstoffen und/oder Additiven, wie Antioxidantien. Nicht enthalten sein müssen insbesondere Polymere, beispielsweise Trägerpolymere, d.h. die Beschichtung ist bevorzugt polymerfrei. In einer anderen Ausführungsform sind Beschichtungen bevorzugt, die nur die Limus-Substanz in kristalliner Form, ggf. in Solvatkristallen, enthalten. In anderen Worten besteht die Beschichtung auf der Ballonoberfläche in dieser anderen bevorzugten Ausführungsform aus der mindestens eine Limus-Substanz in kristalliner Form, ggf. in Solvatkristallen, d.h. es sind -nach Trocknung/Entfernung aller Lösungsmittel- keine weiteren Substanzen vorhanden. In dieser anderen und weiteren anderen bevorzugten Ausführungsformen sind auch insbesondere keine Polymere, beispielsweise Trägerpolymere, vorhanden.

Diese Beschichtungen können auch ohne Zusatz von Antioxidantien über mehr als 1 Jahr bei Raumtemperatur ausreichend stabil sein, d.h. der Wirkstoffstoffgehalt vermindert sich in dieser Zeit um weniger als 5 Gew.-%. Andererseits können verschiedene Hilfsstoffe die Haftung des Wirkstoffs am Ballonmaterial, die Freigabe bei Expansion des Ballons, den Übertritt in die Gefäßwand und die Wirksamkeit und Verträglichkeit positiv beeinflussen. Bevorzugte Hilfsstoffe sind Antioxidantien, bevorzugt in Anteilen von > 5 Gew.-% der Limus-Substanz, hydrophile Substanzen wie Röntgenkontrastmittel, Zucker und Zuckeralkohle, Glycerin, Harnstoff bevorzugt in Gewichtsanteilen von 5-100 Gew.-% der Limus-Substanz, weiterhin amphiphile Substanzen in sehr geringen Anteilen, bevorzugt ≤ 1 Gew.-% der Limus-Substanz und lipophile Substanzen wie Fettsäuresalze bevorzugt im Bereich von 0,5 - 50 Gew.-% der Limus-Substanz.

Arzneilich wirksame Substanzen können als Additive eingesetzt werden.

Die Hilfsstoffe oder Additive, wobei wie oben erwähnt vorzugsweise Polymere ausgenommen sind, können einzeln oder im Gemisch verwendet werden. Wenn im Gemisch verwendet gelten die Mengenangaben für die Summe der Hilfsstoffe bzw. Additive. Die Hilfsstoffe/Additive können der Beschichtungslösung zugesetzt oder bevorzugt vorab auf die Ballonoberfläche aufgetragen werden oder mehr bevorzugt nachträglich, d.h. nach abgeschlossener Trocknung, aufgetragen werden um die Kristallstruktur der Limus-Substanzen nicht zu stören. Werden die Hilfsstoffe/Additive nachträglich aufgetragen, sind Lösungsmittel und Bedingungen zu wählen, die ein Lösen der Limus-Kristalle vermeiden, z.B. Lösungsmittel, in denen die Limus-Substanz schwer löslich ist, leicht flüchtige Lösungsmittel, Spray-Beschichtung, niedrige Temperaturen. Vorzugsweise wird in einem finalen Schritt [c oder e)] mindestens eine zusätzliche Schicht eines Additivs bzw. Hilfsstoffs aufgetragen. Vorzugsweise wird in einer Weise aufgetragen, dass die Limus-Kristalle nicht in eine amorphe Form umgewandelt werden. Alternativ kann gesagt werden, dass in einem zusätzlichen finalen Schritt c oder e) mindestens eine zusätzliche Schicht eines Additivs/Hilfsstoffs ohne Verwendung eines die Limus-Substanz lösenden Mittels aufgetragen wird.

Bei jeder der erfindungsgemäßen Herstellungsvarianten wird bevorzugt polymerfrei gearbeitet und dementsprechend eine polymerfreie Beschichtung erzeugt.

Zur Beschichtung der Ballone sind ansonsten alle gebräuchlichen Methoden möglich, Tauchen, Sprayen, Drucken, Bestreichen, Mikrodosierverfahren etc., bevorzugt sind Mikrodosierverfahren.

Als Ballonmembran kommen alle einsetzbaren formstabilen und dehnbaren Materialien in Frage, insbesondere Polyamide/ Nylon, PEBAX, Polyethylen, Polyurethan, Silikon, Latex, Chronoprene; die Ballonmembranen können zusätzlich durch in den Membranen enthaltene Strukturen (Fäden, Streifen, Drähte) verstärkt werden oder außen von solchen Strukturen umgeben sein wie es beispielsweise bei den, scoring' oder, cutting' Ballonen der Fall ist.

Die Ballone können weiterhin vormontierte ballonexpandierbare oder selbstexpandierende Stents enthalten, die bevorzugt unbeschichtet sind. Die Beschichtung der Ballone erfolgt bevorzugt vor der Montage der Stents, kann aber auch zusätzlich oder allein danach erfolgen.

Die Erfindung wird nachfolgend anhand von Beispielen weitere erläutert, ohne sie auf diese einzuschränken.

### Beispiele

### Beispiel 1

100 mg Everolimus wurden in 1 ml Ethylacetat gelöst. Dann wurden 2 ml Heptan hinzugefügt. Die entstandene Kristall-Suspension wurde mit Ultraschall behandelt und stand damit zur Beschichtung von Ballonkathetern zur Verfügung. Die Beschichtung der Ballone kann im Einzelnen wie oben beschrieben oder wie in den folgenden Beispielen dargestellt erfolgen.

### Beispiel 2

45 mg Sirolimus + 6 mg Butylhydroxytoluol wurden in 0,5 ml Ethylacetat gelöst; danach wurden 0,5 ml Heptan zugesetzt; die Kristallisation des Sirolimus wurde ausgelöst; es entstand eine Mischung einer Sirolimus-Kristallsuspension in gesättigter Sirolimuslösung; die Suspension wurde für 30 min mit Ultraschall behandelt; danach wurde die Suspension auf expandierte Ballone von Kathetern für die perkutane transluminale Koronar-Angioplastie (Sequent^{®}, BBraun) mittels einer Mikrospritze aufgetragen. Nach dem Beschichten wurden die Ballone gefaltet und mittels EO sterilisiert.

Analyse: 6,8 µg Sirolimus/ mm² Ballonoberfläche, Röntgenbeugung und Differential-Thermo-Analyse belegen die kristalline Struktur des Wirkstoffs.

### Beispiel 3

Die Koronararterien von jungen Hausschweinen (ca. 25 kg Körpergewicht) wurden mit Ballonkathetern nach Beispiel 2 behandelt. 2 Tiere (6 behandelte Gefäße) wurden ca. 10 min nach der Behandlung getötet, 11 weitere Tiere (11 behandelte Gefäße) wurden nach 4 Wochen getötet. Bei allen Tieren wurden die behandelten Gefäßsegmente entnommen. Der Sirolimusgehalt der Arterien wurde bestimmt und mit dem Sirolimusgehalt von Arterien der gleichen Tiere verglichen, die mit Ballons gleicher Bauart und Beschichtungszusammensetzung und Dosis (45 mg Sirolimus + 6 mg Butylhydroxytoluol, 7 µg Sirolimus/mm²) behandelt wurden, bei denen Sirolimus jedoch amorph vorlag. Die Ergebnisse sind in Tab. 1 dargestellt. Die kristalline Zubereitung zeigt eine ganz überraschend stark verlängerte Verweildauer im Gewebe: Während die Wirkstoffmenge im Gewebe im Fall der amorphen Zubereitung und identischen Versuchbedingungen innerhalb von 4 Wochen um den Faktor 80 abfiel verminderte sich die Wirkstoffmenge im Fall der kristallin beschichteten Ballone nur um einen Faktor < 3.

Tab. 2 zeigt dass die erfindungsgemäße Formulierung gegenüber dem Stand der Technik zu außerordentlich erhöhten Wirkstoffspiegeln in den Gefäßwänden führt. Solche anhaltend hohen Gewebespiegel werden als entscheidend für die Wirksamkeit in der Restenoseprophylaxe angesehen.

**Tab. 1 Transfer und Verbleib von Sirolimus in die Gefäßwand nach Insufflation beschichteter Angioplastie-Ballone für 1 min in den Koronararterien von Schweinen.**

| Beschichtung | | **Beispiel 2 Sirolimus kristallin** | **Gleiche Zusammensetzung wie Beispiel 2, aber Sirolimus amorph** |
|---|---|---|---|
| Rest-Sirolimus auf benutzten Ballonen (akute Studie), [% der Dosis] | | 2,7 ± 0,8 | 9,2 ± 1,6 |
| | | n=6 | n=6 |
| Rest-Sirolimus auf benutzten Ballonen (4 Wochen Studie) [% der Dosis] | | 2,8 ± 0,6 | 8,9 ± 2,5 |
| | | n=12 | n=12 |
| Sirolimus in der Arterienwand, 10-30 min nach Behandlung | | | |
| | [µg] | 224 ± 52 | 96 ± 64 |
| | [%der Dosis] | 12,5 ± 2,9 | 5,1 ± 3,4 |
| | | n=6 | n=6 |
| Sirolimus in der Arterienwand, 4 Wochen nach Behandlung | | | |
| | [µg] | 83 ± 68 | 1,2 ± 0,9 |
| | [%der Dosis] | 4,7 ± 3,8 | 0,1 ± 0,0 |
| | | n=11 | n=12 |

**Tab. 2 Vergleich mit publizierten Daten: Sirolimus-Konzentration in Koronararterien von Schweinen (ng/mg Gewebe = µg/g Gewebe) nach Behandlung mit Sirolimusbeschichteten Ballonkatheter**

| | **MagicTouch Concept Medical, Inc. Pharm. Liposomen Takimura et al., 2012** | **Caliber Therapeutics, Inc. (nano-particles) Terrez et al., 2012** | **InnoRa/Cordis Sirolimus BHT** US 20100331816 | **Beispiel 2 Sirolimus BHT** | **Gleiche Zusammensetzung wie Beispiel 2; Sirolimus BHT** |
|---|---|---|---|---|---|
| Physikalischer Zustand | unbekannt | unbekannt | unbekannt | kristallin | amorph |
| Stent | nein | nein | ja | ja | ja |
| Zeit nach Behandlung | | | | | |
| sofort | 141 | 423±110 | 313±61 | 547±139 | 219±118 |
| 4 d | | 200±80 | | | |

| | **MagicTouch Concept Medical, Inc. Pharm. Liposomen Takimura et al., 2012** | **Caliber Therapeutics, Inc. (nanoparticles) Terrez et al., 2012** | **InnoRal Cordis Sirolimus BHT** US 20100331816 | **Beispiel 2 Sirolimus BHT** | **Gleiche Zusammensetzung wie Beispiel 2; Sirolimus BHT** |
|---|---|---|---|---|---|
| 7/8 d | 16 | 50±17 | 9,8±10,4 | | |
| 14 d | 6 | | | | |
| 21 d | | 33±14 | | | |
| 28/30 d | | 19±10 | 8,4±5,7 | 136±112 | 2,2±1,8 |
| Mittlere t ½ (0 - 4 Wochen) | < 1 Woche | < 1 Woche | < 1 Woche | > 1 Woche | < 1 Woche |

### Beispiel 4

Koronararterien der Tiere aus Beispiel 3 wurden gleichzeitig mit unbeschichteten Kathetern gleichen Typs behandelt, wobei die Behandlung der Arterien in Bezug auf die Reihenfolge der Katheter und Art der Arterien randomisiert wurde. Unmittelbar nach Behandlung wurde der luminale Durchmesser der leicht überdehnten Koronargefäßsegmente gemessen, nach 4 Wochen wurde die Messung wiederholt. Die Verminderung des Lumendurchmessers während der 4 Wochen wird als late lumen loss (LLL) bezeichnet und kennzeichnet die unerwünschte Verengung der Gefäße durch Neointimaproliferation. Die Ergebnisse finden sich in Tabelle 3.

**Tab. 3 Einfluß der Sirolimus-Beschichtung von Ballonkathetern auf die Verengung von Schweinekoronararterien nach Dehnung/ Gefäßwandverletzung.**

| **Katheter** | **Unbeschichtet** | **Beispiel 2** | **Gleiche Zusammensetzung wie Beispiel 2; Sirolimus BHT amorph** |
|---|---|---|---|
| | | **Sirolimus BHT kristallin** | |
| **Dosis [µg/mm²]** | 0 | 6.8 | 7.1 |
| **n (Gefäße)** | 12 | 11 | 12 |
| **RFD initial [mm]** | 2,62±0,30 | 2,57±0,22 | 2,42±0,19 |
| **MLD post [mm]** | 3,06±0,19 | 2,93±0,32 | 2,88±0,41 |
| **MLD FU [mm]** | 2,36±0,39 | 2,60±0,32 | 2,35±0,43 |
| **LLL [mm]** | 0,70±0,35 | 0,37±0,24* | 0,53±0,52 |
| **Diameter-Stenose** | 23,0%±11,4% | 12,3%±7,8%* | 16,8%±18,9% |
| Dosis = Sirolimus je mm² Ballonoberfläche; RFD = Referenzdurchmesser der Arterie (ohne Behandlung); MLD post = minimaler Lumendurchmesser nach Überdehnung; MLD FU = minimaler Lumendurchmesser nach 4 Wochen; *) p<0,02 versus der unbeschichteten Kontrolle. | | | |

Die mit kristallinem Sirolimus behandelten Gefäße zeigen 4 Wochen nach der Behandlung den größten Lumendurchmesser, den geringsten Lumenverlust und die geringste Diameter-Stenose.

### Referenzbeispiel 5

50 mg Sirolimus wurden in 0,5 ml Ethylacetat gelöst; danach wurden 0,5 ml Heptan zugesetzt. Nach 24 h bei Raumtemperatur hatten sich Kristalle gebildet; die Probe wurde für 30 min mit Ultraschall behandelt; danach wurde die Suspension zentrifugiert, das Sediment einmal mit 1 ml Heptan gewaschen und getrocknet. 5,6 mg Kristalle wurden in 1 ml Heptan suspendiert. Ballone der Größe 2,5 - 20 mm wurden mit 10 µl der Kristallkeimsuspension beschichtet und unmittelbar anschließend mit 43 µl eine Losung von 15 mg Sirolimus in 1 ml Ethylacetat-Heptan (1:1, v/v). Nach kurzer Trocknungszeit wurden homogen weiß beschichtete Ballone erhalten. Sirolimus lag überwiegend kristallin vor.

Die so beschichteten Ballonkatheter wurden mit Stents versehen und in Koronarien junger Schweine wie in den in Beispielen 3 und 4 beschrieben im Hinblick auf die Unterdrückung der Gefäßverengung durch Neointimaproliferation untersucht. Als Vergleich dienten Katheter, deren Ballone nicht beschichtet waren sowie die Beschichtung nach Beispiel 2 bei unterschiedlicher Dosierung.

**Tab. 4 Einfluß der Sirolimus-Beschichtung von Ballonkathetern auf die Verengung von Schweinekoronararterien nach Dehnung/ Gefäßwandverletzung; Vergleich unterschiedlicher Dosierungen und Beschichtungsmethoden**

| **Katheter** | **Unbeschichtet** | **Beispiel 5** | **Beispiel 2** | **Beispiel 2** |
|---|---|---|---|---|
| | | **Sirolimus BHT kristallin** | **Sirolimus BHT kristallin; Dosis reduziert** | **Sirolimus BHT kristallin;** |
| **Dosis [µg/mm²]** | 0 | 3.6±0.5 | 4.0±1.4 | 7.19±0.58 |
| **n (Gefäße)** | 12 | 12 | 13 | 12 |
| **RFD initial [mm]** | 2,61±0,25 | 2,84±0,24 | 2,78±0,20 | 2,74±0,26 |
| **MLD post [mm]** | 3,08±0,35 | 3,19±0,20 | 3,21±0,20 | 3,20±0,22 |
| **MLD FU [mm]** | 2,03±0,58 | 2,53±0,43 | 2,45±0,46 | 2,48±0,51 |
| **LLL [mm]** | 1,05±0,54 | 0,67±0,49 | 0,76±0,48 | 0,72±0,49 |
| **Diameter-Stenose** | 24,3%±16,9% | 9,7%±18,4% | 6,9%±21,1 % | 5,2%± 17,5% |
| Dosis = Sirolimus je mm² Ballonoberfläche; RFD = Referenzdurchmesser der Arterie (ohne Behandlung); MLD post = minimaler Lumendurchmesser nach Überdehnung; MLD FU = minimaler Lumendurchmesser nach 4 Wochen; *) p<0,02 versus der unbeschichteten Kontrolle. | | | | |

Die mit kristallinem Sirolimus behandelten Gefäße zeigen 4 Wochen nach der Behandlung einen größeren Lumendurchmesser, geringeren Lumenverlust und die geringere Diameter-Stenose als die mit unbeschichtetem Ballon behandelten Gefäße.

Kristallines Sirolimus auf Ballonkathetern hemmt reproduzierbar die Verengung der Koronararterien des Schweins nach Gefäßwandverletzung. Die Wirkung wird auch bei deutlich niedrigerer Dosis erzielt als sie in Beispiel 4 benutzt wurde.

### Beispiel 6

Ballone von PTCA-Kathetern (2.5 - 20 mm) wurden wie in Beispiel 5 mit einer geringen Dosis Sirolimus-Impfkristallen beschichtet, danach mit der gennannten Sirolimus-Lösung in Ethylacetat-Heptan und nach dem Trocknen mit 15 µl einer Lösung von 15 mg Probucol/ml Diethylether. Die kristalline Struktur von Sirolimus blieb erhalten.

## Patentansprüche

1. Verfahren zur polymerfreien Beschichtung von Ballonoberflächen von insbesondere Angioplastie-Ballonkatheter mit kristallinen Limus-Substanzen aufweisend die folgenden Schritte:
a) Lösen mindestens einer Limus-Substanz in einem polareren organischen Lösungsmittel,
b) Versetzen der Lösung aus a) mit einem unpolareren organischen Lösungsmittel, so dass entweder eine übersättigte Lösung entsteht oder die echte Löslichkeit erhalten bleibt, anschließend entweder
c) Auftrag der übersättigten oder der echten Lösung auf die Ballonoberfläche und Kristallisation
oder
d) Bei einer übersättigten Lösung Auslösen der Kristallisation der mindestens einen Limus-Substanz, so dass eine kristallhaltige Suspension erhalten wird und
d1) Auftrag der Suspension auf die Ballonoberfläche und weitere Kristallisation und/oder Trocknung.

2. Verfahren zur polymerfreien Beschichtung von Ballonoberflächen von insbesondere Angioplastie-Ballonkatheter mit kristallinen Limus-Substanzen gemäß Anspruch 1, **dadurch gekennzeichnet, dass** in einem zusätzlichen finalen Schritt e) mindestens eine zusätzliche Schicht eines Additivs/Hilfsstoffs aufgetragen wird, wobei Polymere als Additiv/Hilfsstoff ausgenommen sind.

## Claims

1. A method for the polymer-free coating of balloon surfaces, in particular of angioplasty balloon catheters, with crystalline limus substances, comprising the following steps:
a) dissolving at least one Limus substance in a more polar organic solvent,
b) mixing the solution from step a) with a less polar organic solvent so that either a supersaturated solution is formed or the true solubility is maintained, followed by either
c) applying the supersaturated or true solution to the balloon surface and allowing crystallisation
or
d) in the case of a supersaturated solution, initiating the crystallisation of the at least one limus substance so that a crystal-containing suspension is obtained, and
d1) applying the suspension to the balloon surface and further crystallisation and/or drying.

2. The method for the polymer-free coating of balloon surfaces, in particular of angioplasty balloon catheters, with crystalline limus substances according to claim 1, **characterised in that**, in an additional final step e), at least one additional layer of an additive or excipient is applied, polymers being excluded as additives or excipients.

## Revendications

1. Procédé de revêtement sans polymère des surfaces de ballons, en particulier de cathéters à ballonnet d'angioplastie, à l'aide de substances limus cristallines, comprenant les étapes suivantes:
a) dissolution d'au moins une substance de limus dans un solvant organique plus polaire,
b) mélange de la solution de a) avec un solvant organique moins polaire, de manière à obtenir soit une solution sursaturée, soit à conserver la solubilité réelle, puis soit
c) application de la solution sursaturée ou de la solution réelle sur la surface du ballon et cristallisation,
soit
d) dans le cas d'une solution sursaturée, déclenchement de la cristallisation de ladite au moins une substance de limus, de manière à obtenir une suspension contenant des cristaux, puis
d1) application de la suspension sur la surface du ballon et poursuite de la cristallisation et/ou du séchage.

2. Procédé de revêtement sans polymère des surfaces de ballons, en particulier de cathéters à ballonnet d'angioplastie, avec des substances cristallines de type Limus selon la revendication 1, **caractérisé en ce que**, dans une étape finale supplémentaire e), on applique au moins une couche supplémentaire d'un additif ou d'un adjuvant, les polymères étant exclus en tant qu'additifs ou adjuvants.
